# EUROPEAN PATENT SPECIFICATION

(11) **EP 3 603 696 B1**
(45) Date of publication and mention of the grant of the patent: **28.09.2022**
(21) Application number: 18776615.9
(22) Date of filing: 21.02.2018
(51) Int. Cl.: A61M 1/36, A61M 1/16

(54) **BLOOD PURIFICATION DEVICE, CONTROL METHOD THEREOF, AND BLOOD REMOVAL DEFECT ELIMINATION METHOD**
BLUTREINIGUNGSVORRICHTUNG, STEUERUNGSVERFAHREN DAFÜR UND VERFAHREN ZUR BESEITIGUNG VON BLUTENTFERNUNGSDEFEKTEN
DISPOSITIF DE PURIFICATION DU SANG, SON PROCÉDÉ DE COMMANDE ET PROCÉDÉ D'ÉLIMINATION DE DÉFAUT DE PRÉLÈVEMENT DE SANG

(30) Priority: 31.03.2017 JP 2017072412
(43) Date of publication of application: 05.02.2020
(73) Proprietor: Asahi Kasei Medical Co., Ltd., Tokyo 100-0006 (JP)
(72) Inventor: GOTANDA, Yuya, Kawagoe-shi Saitama 350-0833 (JP)
(74) Representative: dompatent von Kreisler Selting Werner - Partnerschaft von Patent- und Rechtsanwälten mbB
(86) International application number: PCT/JP2018/006182
(87) International publication number: WO 2018/180032

(56) References cited:
- EP-A1- 0 309 642
- WO-A1-2016/092913
- GB-A- 2 007 115
- JP-A- 2002 191 691
- JP-A- 2008 023 269
- JP-A- 2013 192 711
- JP-A- 2014 147 793
- JP-A- 2017 006 415

## Description

### Technical Field

The present invention relates to a blood purification device

### Background Art

Blood purification therapies have conventionally been performed by taking out the blood from a patient's body, removing a disease-causing substance, a specific leukocyte, or the like from this blood and returning the resulting blood to the body. In such blood purification therapies, a blood purification device has been used which has a blood circuit for circulating the blood taken out from a body and returning it thereto and a blood purifier equipped with a treating material such as adsorbing material or separating material for removing or separating a specific substance from the blood.

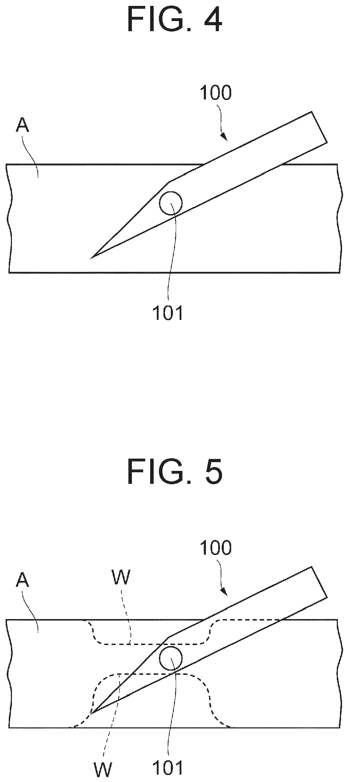

The blood circuit of such a blood purification device is connected to a patient's blood vessel via a puncture needle. For example, a blood removal line of the blood circuit for delivering the blood taken out from a patient to a blood purifier is connected to the artery A of a patient via a puncture needle 100 as shown in Fig. 4. In recent years, there has been proposed an automatic blood return device (refer to, for example, Patent Document 1) for returning the blood which has remained in a blood removal line to a patient via the puncture needle 100 by performing reverse rotation of a blood pump provided in a blood flow path.

EP 309642 discloses a system for the extra corporeal circulation of blood. It is shown that if the pressure drops under a certain value, a valve opens so that venous pressure is increased.

GB 2007115 discloses a blood processing device in which the pressure in said device is monitored and saline is administered to compensate.

### Citation List

### Patent Document

Patent Document 1: Japanese Patent Application Laid-Open No. 2005-224597

### Summary

### Technical Problem

The puncture needle 100 has, at the tip thereof, a hole 101 for drawing the blood thereinto as shown in Fig. 4, but when blood removal (drawing of the blood in the artery A into the blood removal line) is performed via this hole 101 or the tip of the puncture needle 100, an inner wall W of the artery A sometimes adheres to the hole 101 as shown by a broken line in Fig. 5 and causes a blood removal failure. In such a case, when the blood is refluxed by a blood pump as described in Patent Document 1, the hole 101 of the puncture needle 100 is clogged with a clot, which may prevent the blood removal failure from being eliminated.

With the foregoing in view, the present invention has been made. The purpose of the invention is to effectively eliminate, in a blood purification device that performs blood removal via a puncture needle, a blood removal failure resulting from adhesion of a patient's blood vessel to the hole of the puncture needle.

### Solution to Problem

In order to achieve the above-described object, the blood purification device according to the present invention has a blood removal line for delivering the blood taken out from a patient to a blood purifier, a blood pump placed on the blood removal line, a pressure sensor placed in the blood removal line on a side upstream of the blood pump for measuring the pressure of the blood delivered in the blood removal line, a transfusion line for supplying a transfusion to the blood removal line on a side upstream of the blood pump, a transfusion vessel connected to the transfusion line, an opening/closing valve for opening or closing the transfusion line, and a control unit for controlling the blood pump and an opening/closing unit. In the device, the transfusion vessel is placed vertically above the patient and the control unit opens the transfusion line by means of the opening/closing valve when the pressure measured by the pressure sensor becomes smaller than a predetermined value during operation of the blood pump.

By using such a configuration the transfusion line can be opened when the pressure of the blood removal line on a side upstream of the blood pump becomes smaller than a predetermined value during operation of the blood pump (for example, when the blood vessel of a patient adheres to the hole of the puncture needle provided at the end of the blood removal line and causes a blood removal failure). This makes it possible to cause gravity flow of the transfusion in the transfusion vessel, which is placed vertically above the patient, through the transfusion line and supply it to the blood removal line and thereby slightly reflux the blood in the blood removal line to the side of the patient. Thus, the blood removal failure (resulting from, for example, adhesion of the patient's blood vessel to the hole of the puncture needle) can be eliminated effectively. In such a manner, the blood in the blood removal line is refluxed slightly to the side of the patient by making use of a difference in height between the transfusion vessel and the patient without causing a reflux of the blood by the blood pump so that a situation such as clogging of the hole of the puncture needle with the clot can be prevented in advance and a blood removal failure can be eliminated safely.

In the blood purification device of the present invention, the control unit can stop the operation of the blood pump or reduce the speed thereof when a pressure measured by the pressure sensor during operation of the blood pump becomes smaller than a predetermined value.

By using such a configuration, when the pressure of the blood removal line on a side upstream of the blood pump becomes smaller than a predetermined value during operation of the blood pump, operation of the blood pump can be stopped or the speed of it can be reduced. This therefore makes it possible to prevent the transfusion, which is supplied from the transfusion vessel to the blood removal line by gravity flow, from being caused to flow to a side opposite to the patient by the blood pump.

In the blood purification device of the present invention, when the pressure measured by the pressure sensor becomes smaller than a predetermined value during operation of the blood pump, the control unit can stop the operation of the blood pump or reduce the speed thereof, open the transfusion line by means of the opening/closing valve and then, after closing the transfusion line by means of the opening/closing valve, resume the operation of the blood pump at a predetermined flow rate (for example, the flow rate before the operation of the blood pump is stopped or the speed of it is reduced).

By using such a configuration, when the pressure of the blood removal line on a side upstream of the blood pump becomes smaller than a predetermined value during operation of the blood pump, a blood removal failure can be eliminated by stopping the operation of the blood pump or reducing the speed thereof and opening the transfusion line. Then, the transfusion line is closed and the operation of the blood pump can be automatically started again at a predetermined flow rate.

In the blood purification device of the present invention, the transfusion line or the blood removal line between the patient and the transfusion line can be provided with an air bubble detector. In such a case, when the air bubble sensor detects air bubbles when the transfusion line is opened by means of the opening/closing valve, the control unit can close the transfusion line by means of the opening/closing valve.

By using such a configuration, when the transfusion line is opened to eliminate a blood removal failure and the air bubble detector provided in the transfusion line (or the blood removal line positioned between the patient and the transfusion line) detects air bubbles, the transfusion line can be closed. This therefore makes it possible to prevent air bubbles from being contained in the blood to be refluxed to the side of the patient or in the transfusion.

In the blood purification device of the present invention, the control unit can open the transfusion line only for a predetermined time by means of the opening/closing valve.

By using such a configuration, when the pressure of the blood removal line on a side upstream of the blood pump becomes smaller than a predetermined value during operation of the blood pump, it is possible to stop the operation of the blood pump and open the transfusion line only for a predetermined time (for example, for 1 to 2 seconds). In short, the open state of the transfusion line can be controlled based on time.

In the blood purification device of the present invention, the control unit can open the transfusion line by means of the opening/closing valve until the pressure measured by the pressure sensor reaches a predetermined value or more.

By using such a constitution, when the pressure of the blood removal line on a side upstream of the blood pump becomes smaller than a predetermined value during operation of the blood pump, it is possible to stop the operation of the blood pump and open the transfusion line until the pressure measured by the pressure sensor reaches a predetermined value or more. In short, the open state of the transfusion line can be controlled based on a pressure.

### Advantageous Effects of Invention

The present invention makes it possible to effectively eliminate, in a blood purification device for performing blood removal via a puncture needle, a blood removal failure resulting from adhesion of a patient's blood vessel to the hole of the puncture needle.

### Brief Description of Drawings

Fig. 1 is a block diagram for describing the entire constitution of a blood purification device according to an embodiment of the present invention.
Fig. 2 is a view showing a transfusion line closed
Fig. 3 is a view showing the transfusion line opened
Fig. 4 is an enlarged view of a puncture needle provided at the end of a blood removal line of a blood purification device.
Fig. 5 is a view showing occurrence of a blood removal failure due to adhesion of a blood vessel to the hole of the puncture needle shown in Fig. 4.

### Description of Embodiments

Embodiments of the present invention will hereinafter be described referring to the drawings.

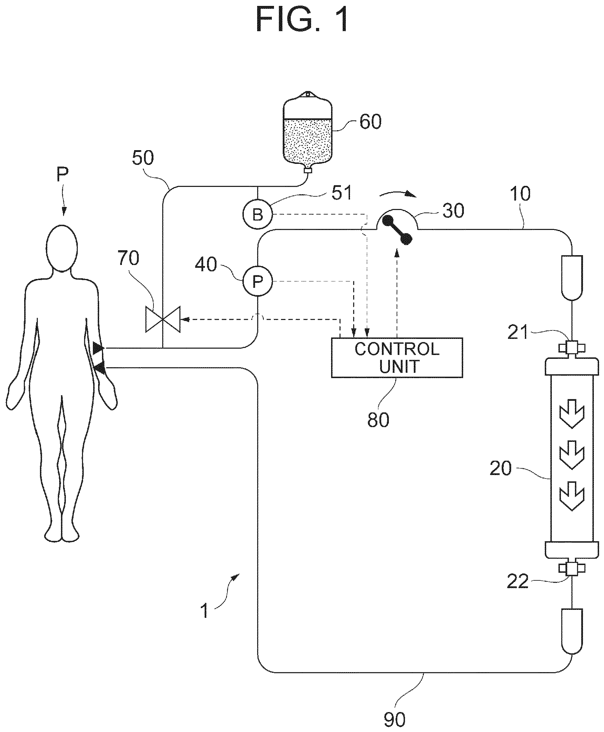

First, the constitution of a blood purification device 1 according to the embodiment of the present invention is described referring to Fig. 1.

The blood purification device 1 according to the present embodiment is used for so-called leukocyte apheresis (LCAP) and as shown in Fig. 1, it is equipped with a blood removal line 10, a blood purifier 20, a blood pump 30, a pressure sensor 40, a transfusion line 50, a transfusion vessel 60, an opening/closing valve 70, a control unit 80, a blood return line 90, and the like.

The blood removal line 10 is a flow path for delivering the blood taken out from a patient P to the blood purifier 20, while the blood return line 90 is a flow path for returning the blood purified by the blood purifier 20 to the patient P. The blood removal line 10 and the blood return line 90 constitute a blood circuit for circulating the blood taken out from the body of the patient P and returning it into the body. The blood removal line 10 has, at the end thereof, a puncture needle 100 as shown in Fig. 4 and via this puncture needle 100, the blood removal line 10 is connected to the artery of the patient P. Similarly, the blood return line 90 is connected to the vein of the patient P via a puncture needle not shown in the drawing.

The blood purifier 20 purifies the blood introduced from a blood inlet 21 by removing an activated leukocyte contained in the blood through a filter and discharges the purified blood from a blood outlet 22. As the filter, that having an adsorbing material for removing the activated leukocyte can be used.

The blood pump 30 is placed on the blood removal line 10 as shown in Fig. 1 and functions to deliver the blood of the patient P to the blood purifier 20 via the blood removal line 10 by performing operation (forward rotation) under the control of the control unit 80. When the control unit 80 stops the operation of the blood pump 30, on the other hand, the delivery of the blood in the blood removal line 10 is stopped accordingly.

The pressure sensor 40 is placed in the blood removal line 10 on a side upstream of the blood pump 30 and functions to measure the pressure of the blood delivered in the blood removal line 10. The pressure data measured by the pressure sensor 40 is sent to the control unit 80 and used for the control of the blood pump 30 and the opening/closing valve 70.

The transfusion line 50 is a flow path for supplying a transfusion to the blood removal line 10 on a side upstream of the blood pump 30. The transfusion line 50 in the present embodiment is equipped with an air bubble detector 51 for detecting air bubbles contained in the transfusion delivered in the transfusion line 50. The information relating to the results detected by the air bubble detector 51 is sent to the control unit 80 and used for the control of the opening/closing valve 70.

The transfusion vessel 60 is a vessel for accumulating a transfusion therein and it is connected to the end of the transfusion line 50. The transfusion vessel 60 in the present embodiment is placed vertically above the patient P so that when the transfusion line 50 is opened by means of the opening/closing valve 70, the transfusion moves by gravity flow from the transfusion vessel 60 to the side of the patient P. As the transfusion, physiological saline or the like can be used.

The opening/closing valve 70 is placed on the transfusion line 50 as shown in Fig. 1 and functions to work under the control of the control unit 80 and open or close the transfusion line 50. When the transfusion line 50 is opened by means of the opening/closing valve 70, the transfusion flows from the transfusion vessel 60 to the blood removal line 10 via the transfusion line 50 and by this transfusion, the blood which has accumulated in the blood removal line 10 is returned to the side of the patient P. As a result, a blood removal failure can be eliminated.

The control unit 80 is comprised of, for example, a computer equipped with a memory and CPU (Central Processing Unit) and various programs recorded in the memory are performed by CPU to control various units of the blood purification device 1.

More specifically, when a pressure measured by the pressure sensor 40 becomes smaller than a predetermined value (for example, -200 mmHg) during operation of the blood pump 30, the control unit 80 controls the blood pump 30 and the opening/closing valve 70 so as to stop the operation of the blood pump 30 and open the transfusion line 50 by means of the opening/closing valve 70 for a predetermined time (for example, for 1 to 2 seconds). At this time, as soon as the transfusion line 50 is opened by means of the opening/closing valve 70 and air bubbles are detected by the air bubble detector 51, the control unit 80 closes the transfusion line 50 by means of the opening/closing valve 70. Then, the control unit 80 closes the transfusion line 50 by means of the opening/closing valve 70 and resume the operation of the blood pump 30 at a predetermined flow rate (for example, a flow rate before the blood pump 30 is stopped).

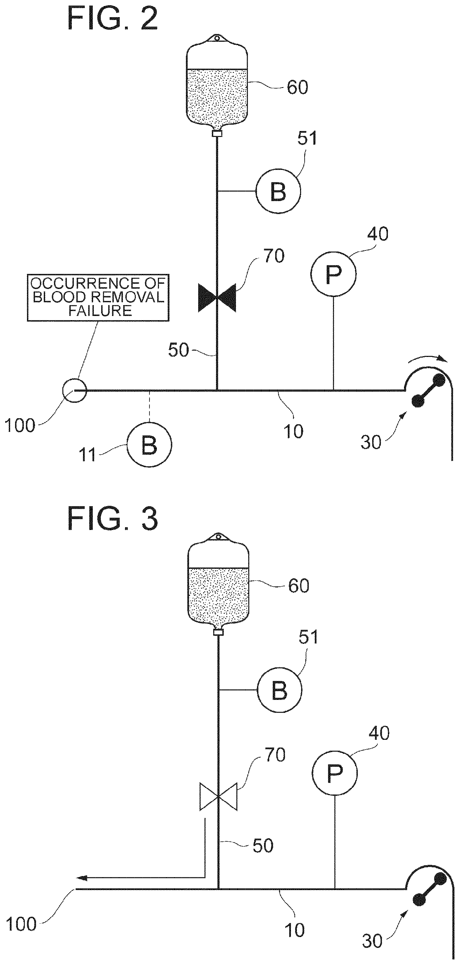

First, as shown in Fig. 2, the control unit 80 measures the pressure of the blood removal line 10 by the pressure sensor 40 while closing the transfusion line 50 by means of the opening/closing valve 70 and at the same time operating the blood pump 30, and judges that a blood removal failure has occurred when the measured pressure becomes smaller than a predetermined value (for example, -200 mmHg). Such a blood removal failure occurs, for example, because the blood vessel of the patient P adheres to the hole 101 of the puncture needle 100 as shown in Fig. 5.

After detection of the blood removal failure, **as shown in** **Fig. 3****,** the control unit 80 controls the blood pump 30 and the opening/closing valve 70 so as to stop the operation of the blood pump 30 and at the same time, open the transfusion line 50 for a predetermined time (for example, for 1 to 2 seconds) by means of the opening/closing valve 70. When the transfusion line 50 is opened by means of the opening/closing valve 70, the transfusion flows from the transfusion vessel 60 to the blood removal line 10 via the transfusion line 50 and by this transfusion, the blood which has accumulated in the blood removal line 10 is returned to the side of the patient P. As a result, the blood removal failure is eliminated.

As soon as the control unit 80 opens the transfusion line 50 by means of the opening/closing valve 70 and the air bubble detector 51 detects air bubbles, the control unit 80 closes the transfusion line 50 by means of the opening/closing valve 70. By this, the blood (or transfusion) to be refluxed to the side of the patient P can be protected from containing air bubbles. Then, the control unit 80 closes the transfusion line 50 by means of the opening/closing valve 70 and resumes the operation of the blood pump 30 at a predetermined flow rate.

In the blood purification device 1 according to the embodiment described above, when the pressure of the blood removal line 10 on a side upstream of the blood pump 30 becomes smaller than a predetermined value during operation of the blood pump 30 (for example, when the blood vessel of the patient P adheres to the hole 101 of the puncture needle 100 provided at the end of the blood removal line 10 and causes a blood removal failure), the operation of the blood pump 30 can be stopped and the transfusion line 50 can be opened. This makes it possible to cause gravity flow, via the transfusion line 50, of the transfusion in the transfusion vessel 60 placed vertically above the patient P, supply it to the blood removal line 10, and cause slight reflux of the blood in the blood removal line 10 to the side of the patient P. A blood removal failure (due to, for example, adhesion of the blood vessel of the patient P to the hole 101 of the puncture needle 100) can therefore be eliminated effectively. Since the blood in the blood removal line 10 is thus slightly refluxed to the side of the patient P by making use of a difference in height between the transfusion vessel 60 and the patient P without refluxing the blood by the blood pump 30, a situation such as clogging of the hole 101 of the puncture needle 100 with a clot can be prevented in advance and the blood removal failure can be eliminated safely.

In the blood purification device 1 according to the embodiment described above, when the transfusion line 50 is opened in order to eliminate a blood removal failure and the air bubble detector 51 provided in the transfusion line 50 detects air bubbles, the transfusion line 50 can be closed. Therefore, the blood (or transfusion) to be refluxed to the side of the patient P can be protected from containing air bubbles.

In the blood purification device 1 according to the embodiment described above, when the pressure of the blood removal line 10 on a side upstream of the blood pump 30 becomes smaller than a predetermined value during operation of the blood pump 30, it is possible to stop the operation of the blood pump 30 and open the transfusion line 50 only for a predetermined time (for example, for 1 to 2 seconds). In short, the open state of the transfusion line 50 can be controlled based on time.

In the blood purification device 1 according to the embodiment described above, when the pressure of the blood removal line 10 on a side upstream of the blood pump 30 becomes smaller than a predetermined value during operation of the blood pump 30, a blood removal failure can be eliminated by stopping the operation of the blood pump 30 and opening the transfusion line 50. Then, the transfusion line 50 is closed and the operation of the blood pump 30 can be resumed automatically.

In the present embodiment, an example of providing the transfusion line 50 with the air bubble detector 51 is shown. Alternatively, as shown by the broken line in Fig. 2, an air bubble detector 11 may be provided in the blood removal line 10 positioned between the patient P and the transfusion line 50.

As the control unit 80 of the present embodiment, shown is an example of a control unit that opens the transfusion line 50 for a predetermined time by means of the opening/closing valve 70 (an example of controlling the open state of the transfusion line 50 based on time) when the pressure measured by the pressure sensor 40 becomes smaller than a predetermined value. The control mode of the opening/closing valve 70 is however not limited to it. For example, when the pressure measured by the pressure sensor 40 becomes smaller than a predetermined value, the control unit 80 may open the transfusion line 50 by means of the opening/closing valve 70 until the pressure measured by the pressure sensor 40 reaches a predetermined value (for example, 0 mmHg) or more (meaning that the open state of the transfusion line 50 is controlled based on a pressure).

As the control unit 80 of the present embodiment, shown is an example of a control unit that opens the transfusion line 50 after stopping the operation of the blood pump 30 when the pressure measured by the pressure sensor 40 becomes smaller than a predetermined value. When the flow rate of the blood pump 30 is relatively small, the transfusion line 50 may be opened after continuing the operation of the blood pump 30 (or after reducing the speed of the pump without completely stopping it).

In the present embodiment, shown is an application example of the present invention to a blood purification device used for leukocyte apheresis (LCAP). The present invention can also be applied to another blood purification device having a similar constitution (puncture needle, blood removal line, blood pump, pressure sensor, transfusion line, transfusion vessel, and opening/closing valve). The present invention can also be applied to a blood purification device equipped with a blood purifier for filtration or diffusion to be used for, for example, plasma exchange (PE), double filtration plasmapheresis (DFPP), continuous hemofiltration (CHF), continuous hemodialysis (CHD), or continuous hemodiafiltration (CHDF).

### [Reference Signs List]

1: Blood purification device
10: Blood removal line
11: Air bubble detector
20: Blood purifier
30: Blood pump
40: Pressure sensor
50: Transfusion line
51: Air bubble detector
60: Transfusion vessel
70: Opening/closing valve
80: Control unit
P: Patient

## Claims

1. A blood purification device (1) comprising: a blood removal line (10) for delivering a blood taken out from a patient (P) to a blood purifier (20); a blood pump (30) placed on the blood removal line (10); a pressure sensor (40) placed in the blood removal line (10) on a side upstream of the blood pump (30) for measuring a pressure of the blood delivered in the blood removal line (10); a transfusion line (50) for supplying a transfusion to the blood removal line (10) on a side upstream of the blood pump (30); a transfusion vessel (60) connected to the transfusion line (50); an opening/closing valve (70) for opening or closing the transfusion line (50); and a control unit (20) for controlling the blood pump (30) and the opening/closing valve (70), wherein:
the transfusion vessel (60) is placed vertically above the patient (P); and
the control unit (80) opens the transfusion line (50) by means of the opening/closing valve (70) when the pressure measured by the pressure sensor (40) becomes smaller than a predetermined value during operation of the blood pump (30).

2. The blood purification device (1) according to Claim 1, wherein the control unit (80) stops the operation of the blood pump (30) or reduces a speed thereof when the pressure measured by the pressure sensor (40) becomes smaller than a predetermined value during operation of the blood pump (30).

3. The blood purification device (1) according to Claim 1, wherein when the pressure measured by the pressure sensor (40) becomes smaller than a predetermined value during operation of the blood pump (30), the control unit (80) stops the operation of the blood pump (30) or reduces a speed thereof and opens the transfusion line (50) by means of the opening/closing valve (70) and then, closes the transfusion line by means of the opening/closing valve and resumes the operation of the blood pump (30) at a predetermined flow rate.

4. The blood purification device (1) according to Claim 3, wherein the predetermined flow rate is a flow rate before the control unit (80) stops the operation of the blood pump (30) or reduces the speed thereof.

5. The blood purification device according to any one of Claims 1 to 4, wherein:
an air bubble detector (11) is provided in the transfusion line (50) or in the blood removal line (10) positioned between the patient (P) and the transfusion line (50); and
the control unit (80) closes the transfusion line (50) by means of the opening/closing valve (70) when the transfusion line (50) is opened by means of the opening/closing valve (70) and the air bubble detector (11) detects an air bubble.

6. The blood purification device (1) according to any one of Claims 1 to 5, wherein the control unit (80) opens the transfusion line (50) for a predetermined time by means of the opening/closing valve (70).

7. The blood purification device (1) according to any one of Claims 1 to 5, wherein the control unit (80) opens the transfusion line (50) by means of the opening/closing valve (70) until the pressure measured by the pressure sensor (40) reaches the predetermined value or more.

## Patentansprüche

1. Blutreinigungsvorrichtung (1), umfassend: eine Blutentnahmeleitung (10) zum Abgeben von Blut, das einem Patienten (P) entnommen wurde, an einen Blutreiniger (20); eine Blutpumpe (30), die in die Blutentnahmeleitung (10) geschaltet ist; einen Drucksensor (40), der in der Blutentnahmeleitung (10) der Blutpumpe (30) vorgeschaltet ist, um den Druck des abgegebenen Bluts in der Blutentnahmeleitung (10) zu messen; eine Transfusionsleitung (50) zum Zuführen einer Transfusion zu der Blutentnahmeleitung (10) auf einer Seite vor der Blutpumpe (30); ein Transfusionsgefäß (60), das an die Transfusionsleitung (50) angeschlossen ist; ein Öffnungs-/Schließventil (70) zum Öffnen oder Schließen der Transfusionsleitung (50); und eine Steuereinheit (20) zum Steuern der Blutpumpe (30) und des Öffnungs-/Schließventils (70), wobei:
das Transfusionsgefäß (60) vertikal über dem Patienten (P) platziert wird; und
die Steuereinheit (80) mittels des Öffnungs-/Schließventils (70) die Transfusionsleitung (50) öffnet, wenn der durch den Drucksensor (40) gemessene Druck während des Betriebs der Blutpumpe (30) kleiner wird als ein vorbestimmter Wert.

2. Blutreinigungsvorrichtung (1) gemäß Anspruch 1, wobei die Steuereinheit (80) den Betrieb der Blutpumpe (30) abbricht oder deren Geschwindigkeit reduziert, wenn der durch den Drucksensor (40) gemessene Druck während des Betriebs der Blutpumpe (30) kleiner wird als ein vorbestimmter Wert.

3. Blutreinigungsvorrichtung (1) gemäß Anspruch 1, wobei dann, wenn der durch den Drucksensor (40) gemessene Druck während des Betriebs der Blutpumpe (30) kleiner wird als ein vorbestimmter Wert, die Steuereinheit (80) den Betrieb der Blutpumpe (30) abbricht oder deren Geschwindigkeit reduziert und mittels des Öffnungs-/Schließventils (70) die Transfusionsleitung (50) öffnet und dann mittels des Öffnungs-/Schließventils die Transfusionsleitung schließt und den Betrieb der Blutpumpe (30) mit einer vorbestimmten Fließgeschwindigkeit wieder aufnimmt.

4. Blutreinigungsvorrichtung (1) gemäß Anspruch 3, wobei die vorbestimmte Fließgeschwindigkeit der Fließgeschwindigkeit entspricht, bevor die Steuereinheit (80) den Betrieb der Blutpumpe (30) abbricht oder deren Geschwindigkeit reduziert.

5. Blutreinigungsvorrichtung gemäß einem der Ansprüche 1 bis 4, wobei:
in der Transfusionsleitung (50) oder in der Blutentnahmeleitung (10) ein Luftblasendetektor (11) vorhanden ist und sich zwischen dem Patienten (P) und der Transfusionsleitung (50) befindet; und
die Steuereinheit (80) mittels des Öffnungs-/Schließventils (70) die Transfusionsleitung (50) schließt, wenn die Transfusionsleitung (50) mittels des Öffnungs-/Schließventils (70) geöffnet ist und der Luftblasendetektor (11) eine Luftblase erkennt.

6. Blutreinigungsvorrichtung (1) gemäß einem der Ansprüche 1 bis 5, wobei die Steuereinheit (80) mittels des Öffnungs-/Schließventils (70) die Transfusionsleitung (50) während einer vorbestimmten Zeit öffnet.

7. Blutreinigungsvorrichtung (1) gemäß einem der Ansprüche 1 bis 5, wobei die Steuereinheit (80) mittels des Öffnungs-/Schließventils (70) die Transfusionsleitung (50) öffnet, bis der durch den Drucksensor (40) gemessene Druck den vorbestimmten Wert erreicht oder übertrifft.

## Revendications

1. Dispositif de purification du sang (1) comprenant: une ligne de retrait du sang (10) pour transmettre un sang prélevé d'un patient (P) à un purificateur de sang (20); une pompe à sang (30) placée sur la ligne de retrait du sang (10); un capteur de pression (40) placé dans la ligne de retrait du sang (10) sur un côté en amont de la pompe à sang (30) pour mesurer une pression du sang transmis dans la ligne de retrait du sang (10); une ligne de transfusion (50) pour fournir une transfusion à la ligne de retrait du sang (10) sur un côté en amont de la pompe à sang (30); un récipient de transfusion (60) relié à la ligne de transfusion (50); une vanne d'ouverture/fermeture (70) pour ouvrir ou fermer la ligne de transfusion (50); et une unité de commande (20) pour commander la pompe à sang (30) et la vanne d'ouverture/fermeture (70), dans lequel:
le récipient de transfusion (60) est placé verticalement au-dessus du patient (P); et
l'unité de commande (80) ouvre la ligne de transfusion (50) au moyen de la vanne d'ouverture/fermeture (70) lorsque la pression mesurée par le capteur de pression (40) devient inférieure à une valeur prédéterminée pendant le fonctionnement de la pompe à sang (30).

2. Dispositif de purification du sang (1) selon la revendication 1, dans lequel l'unité de commande (80) arrête le fonctionnement de la pompe à sang (30) ou réduit une vitesse de celle-ci lorsque la pression mesurée par le capteur de pression (40) devient inférieure à une valeur prédéterminée pendant le fonctionnement de la pompe à sang (30).

3. Dispositif de purification du sang (1) selon la revendication 1, dans lequel lorsque la pression mesurée par le capteur de pression (40) devient inférieure à une valeur prédéterminée pendant le fonctionnement de la pompe à sang (30), l'unité de commande (80) arrête le fonctionnement de la pompe à sang (30) ou réduit une vitesse de celle-ci et ouvre la ligne de transfusion (50) au moyen de la vanne d'ouverture/fermeture (70) et ensuite ferme la ligne de transfusion au moyen de la vanne d'ouverture/fermeture et reprend le fonctionnement de la pompe à sang (30) à un débit prédéterminé.

4. Dispositif de purification du sang (1) selon la revendication 3, dans lequel le débit prédéterminé est un débit avant que l'unité de commande (80) arrête le fonctionnement de la pompe à sang (30) ou réduise la vitesse de celle-ci.

5. Dispositif de purification du sang selon l'une quelconque des revendications 1 à 4, dans lequel:
un détecteur de bulles d'air (11) est prévu dans la ligne de transfusion (50) ou dans la ligne de retrait du sang (10) positionné entre le patient (P) et la ligne de transfusion (50); et
l'unité de commande (80) ferme la ligne de transfusion (50) au moyen de la vanne d'ouverture/fermeture (70) lorsque la ligne de transfusion (50) est ouverte au moyen de la vanne d'ouverture/fermeture (70) et le détecteur de bulles d'air (11) détecte une bulle d'air.

6. Dispositif de purification du sang (1) selon l'une quelconque des revendications 1 à 5, dans lequel l'unité de commande (80) ouvre la ligne de transfusion (50) pendant un temps prédéterminé au moyen de la vanne d'ouverture/fermeture (70).

7. Dispositif de purification du sang (1) selon l'une quelconque des revendications 1 à 5, dans lequel l'unité de commande (80) ouvre la ligne de transfusion (50) au moyen de la vanne d'ouverture/fermeture (70) jusqu'à ce que la pression mesurée par le capteur de pression (40) atteigne la valeur prédéterminée ou plus.
